Europäisches Patentamt

European Patent Office

Office européen des brevets

(19)

(11) Publication number: **0 289 681 B1**

(12) **EUROPEAN PATENT SPECIFICATION**

(45) Date of publication of patent specification: **25.09.91**  (51) Int. Cl.⁵: **A61F 13/04**, A61L 15/07

(21) Application number: **87310986.2**

(22) Date of filing: **14.12.87**

(54) **An orthopaedic cast.**

(30) Priority: **27.04.87 US 43201**

(43) Date of publication of application:
**09.11.88 Bulletin 88/45**

(45) Publication of the grant of the patent:
**25.09.91 Bulletin 91/39**

(84) Designated Contracting States:
**AT BE CH DE ES FR GB GR IT LI LU NL SE**

(56) References cited:
**GB-A- 1 335 123**
**US-A- 3 882 857**
**US-A- 4 194 041**
**US-A- 4 238 522**

(73) Proprietor: **W.L. GORE & ASSOCIATES, INC.**
**555 Paper Mill Road P.O. Box 9329**
**Newark Delaware 19714-9206(US)**

(72) Inventor: **Norvell, Jean**
**30 Park Drive Silverbrook**
**Newark Delaware 19713(US)**

(74) Representative: **McCallum, William Potter et al**
**Cruikshank & Fairweather 19 Royal Exchange Square**
**Glasgow G1 3AE Scotland(GB)**

## Description

This invention relates to an orthopaedic cast of the type used to immobolize a fractured bone while it heals.

In applying a conventional plaster-of-paris cast, it is standard practice to first cover the body member being treated with a tubular protective cloth bandage or wrap, such as cotton or polyester knit fabric. The cloth tube-covered body member is then covered with cotton or polyester padding to provide a soft resilient padded protective liner. This padding is then overwrapped with wetted plaster-of-paris, in woven cloth wrapping of sufficient length and to such a thickness, so as to provide an immobilizing structure of adequate strength and durability for the intended length of its stay in place on the body member being treated.

It is also known to make casts from glass fibre materials, which consist of heat-softened or curable organic polymeric resins coated on glass fibres which have been woven into fabrics or are formed into unwoven open fibrous mats. Such flexible coated glass fabrics are wrapped while warm about a body member to be immobilized in much the same manner as plaster-containing cloth and allowed to cool and harden after the cast is formed. U.S. Patent 3,882,857, describes a resin/glass fibre composite method as do U.S. Patents 4,273,115 and 4,238,522.

While the conventional plaster-of-paris casting system and the glass fibre casting system are in widespread use, they have several disadvantages. Both types of cast are considered in the art to be non-breathable and both must be kept dry inside the cast since they cannot breathe.

Although the casts themselves are impervious to water and not affected by immersion in water, the skin is wet under the cast. A lack of air to dry the skin could permit maceration of the skin under the cast, thus opening it to attack by bacteria. An additional problem with many casts is that odour develops owing to retention of perspiration and body oils in the cast.

In U.S. 3882857 the skin is dried by air circulation through the cast.

The present invention provides an immobilizing orthopaedic cast comprising: an inner, generally tubular, protective sleeve adjacent the skin, made of a composite membrane comprising a liquid-water-impermeable water-vapour-permeable polymeric membrane layer having a hydrophilic layer attached thereto; a layer of resilient padding extending around said sleeve; and an outer immobilizing layer extending around said padding.

An embodiment of the invention will now be described by way of example only.

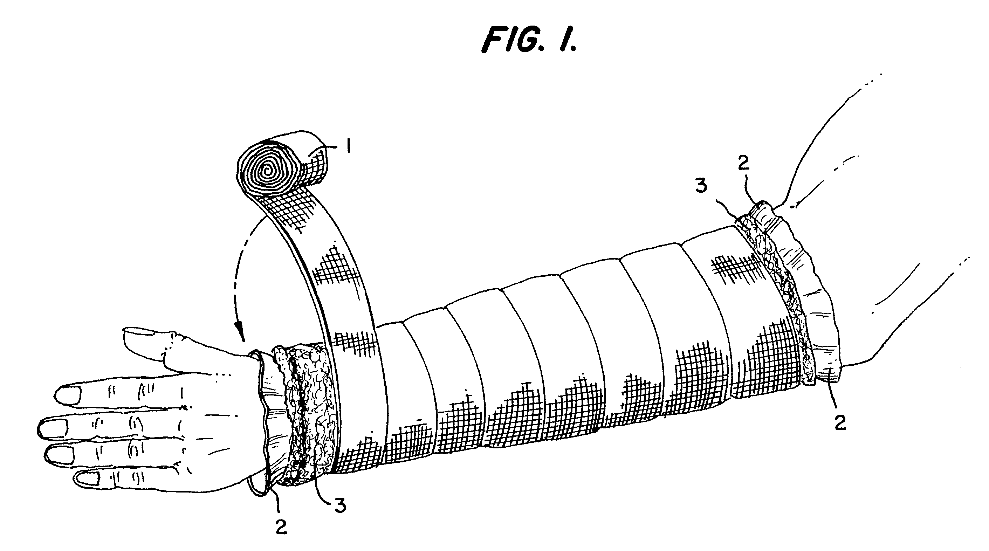

The invention is illustrated in the accompanying drawing of which the single figure diagrammatically shows a human arm over which a tubular sleeve according to the invention has been drawn and a roll of plaster or plastic resin/glass fibre wrap is being wound around the arm and sleeve.

The cast of the illustrated embodiment comprises an inner protective sleeve 2 of approximately cylindrical configuration to be worn against the skin, over which a layer 3 of soft, resilient cotton or polyester padding is wound, folded, or otherwise formed into a protective layer. Over both of these layers an immobilizing cylinder of plaster-of-paris or organic polymer resin/glass fibre composite splinting material is formed, such as by winding a wet plaster-of-paris bandage (1) as shown.

The inner protective sleeve 2 is a heat-sealed wrapper of a porous liquid water impenetrable, water-vapour-permeable organic polymeric membrane, preferably of porous expanded polytetrafluoroethylene. The material of this sleeve permits water, perspiration, or urine to evaporate from the skin through the membrane by moisture vapour transmission so that the skin remains dry. Moisture vapour transmission of the membrane is the operative mechanism here, not air permeability. Preferably the sleeve is formed from a composite membrane which has a continuous hydrophilic layer that transmits water by an absorption-evaporation mechanism, the hydrophilic layer being attached to a microporous hydrophobic polymer layer which permits no detectable passage of liquid water up to 100 pounds per square inch (689.5kPa) pressure. Any known membrane having the requisite properties may be used for this layer of the cast although the preferred materials are the microporous expanded polytetrafluoroethylene membranes described in U.S. Patents 4,194,041 and 4,443,511. Also utilisable are microporous polypropylene films and polyurethane films and tightly woven fabrics of polyolefin fibres or polytetrafluoroethylene or other fluorinated polymer fibres.

Breathability is achieved by evaporation of water inside the inner surface of the membrane, followed by gaseous diffusion of water vapour through the membrane to the outside.

This ensures that the surface of the skin remains dry, thus guarding against immersion skin and skin irritation. Because of the intrinsic pore size of the membrane (9 billion per square inch), the membrane also serves to deter bacterial development, since it is a natural barrier to bacteria which cannot pass through pores of such small size. Also, the membrane has no nutritive substance for bacteria to feed upon. Thus, odour caused by rampant bacterial growth on moist skin unable to dry will not occur. Body secretions or wound drainage cannot seep into padding or ultimately into the cast itself. Therefore odour is greatly reduced.

In placing the heat sealed sleeve 2 over the area to be immobilised and overwrapped, the small ends of membrane sleeve 2 are left uncovered. These may later be trimmed off after the cast has been completely formed and cured or may be folded up over a first layer of the wetted cloth/plaster-of-paris web composite wrapping 1 which is now wrapped around the padded, membrane-covered area to be immobilized and anchored or buried inside layers of the wrapping. The cast is completed when an immobilizing thickness of material has been added and it has hardened sufficiently. Any liquid water which might enter the plaster layer of the cast during wear cannot of course penetrate the protective membrane sleeve 2 to harm the wearer and will evaporate through the padding or the outer surface of the cast. Most plaster casts are also air permeable as well.

Alternatively to the water-wet cloth/plaster-of-paris immobilizing layer, an organic polymer resin-impregnated or coated glass fibre cloth, roving, non-woven mesh, mat, or other glass or plastic fibre material in appropriately sized strips may be wound around the padding layer 3 and the protective sleeve 2 to form an immobilizing orthopaedic cast. Many of these materials are warmed to levels above room temperature but to heat levels well tolerable by the body, and while soft, are wound, formed or shaped into a cast which then hardens fairly rapidly to an immobilizing stiffness. These resin/glass fibre casts are usually waterproof in practice, transmit little water vapour, and are not air breathable.

## Claims

1. An immobilizing orthopaedic cast comprising: an inner, generally tubular, protective sleeve (2) adjacent the skin, made of a composite membrane comprising a liquid-water-impermeable water-vapour-permeable polymeric membrane layer having a hydrophilic layer attached thereto; a layer of resilient padding (3) extending around said sleeve; and an outer immobilizing layer (1) extending around said padding.

2. A cast according to claim 1 wherein the liquid-water-impermeable water-vapour-permeable membrane layer is a microporous organic polymeric membrane.

3. A cast according to claim 2, wherein said membrane is made of expanded polytetrafluoroethylene.

4. A cast according to any preceding claim wherein the sleeve (2) has been formed from membrane sheet material by heat-sealing together adjacent edges of the sheet.

5. A cast according to any preceding claim wherein said immobilizing layer (1) comprises one or more layers of plaster splinting material.

6. A cast according to any one of claims 1 to 4 wherein said immobilizing layer (1) comprises an organic polymeric material containing a reinforcing material.

7. A cast according to claim 6, wherein the reinforcing material comprises glass fibres or organic polymeric fibres.

## Revendications

1. Moulage orthopédique immobilisant qui comprend: un manchon protecteur interne sensiblement tubulaire (2) adjacent à la peau, confectionné en une membrane composite comprenant une couche de membrane polymère imperméable à l'eau liquide mais perméable à la vapeur d'eau présentant une couche hydrophile fixée sur elle ; une couche de molleton flexible (3) s'étendant autour dudit manchon ; et une couche immobilisante externe (1) s'étendant autour dudit molleton .

2. Moulage selon la revendication 1, dans lequel la couche de membrane imperméable à l'eau liquide mais perméable à la vapeur d'eau est une membrane polymère organique microporeuse.

3. Moulage selon la revendication 2, dans lequel ladite membrane est confectionnée en polytetrafluoréthylène expansé.

4. Moulage selon l'une quelconque des revendications précédentes dans lequel le manchon (2) a été formé à partir d'une feuille de membrane par thermoscellement de jonction des bords adjacents de la feuille.

5. Moulage selon l'une quelconque des revendications précédentes, dans lequel ladite couche immobilisante (1) comprend une ou plusieurs couche(s) de matériau d' attelle en plâtre.

6. Moulage selon l'une quelconque des revendications 1 à 4 dans lequel ladite couche immobilisante (1) comprend une matière polymère organique contenant un matériau de renforcement.

7.  Moulage selon la revendication 6, dans lequel le matériau de renforcement comprend des fibres de verre ou des fibres polymères organiques.

**Patentansprüche**

1.  Unbeweglich-machender orthopädischer Verband, umfassend: eine der Haut benachbarte innere, etwa rohrförmige Schutzmanschette (2), hergestellt aus einer zusammengesetzten Membran, die eine für flüssiges Wasser undurchdringliche und für Wasserdampf durchlässige polymere Membranschicht aufweist, an der eine hydrophile Schicht befestigt ist; eine sich um die Manschette herum erstreckende Schicht aus einer elastischen Füllung (3); und eine äußere, unbeweglich-machende Schicht (1), die sich um die Füllung herum erstreckt.

2.  Verband nach Anspruch 1, bei dem die für flüssiges Wasser undurchdringliche und für Wasserdampf durchlässige Membranschicht eine mikroporöse, organische polymere Membran ist.

3.  Verband nach Anspruch 2, bei dem die Membran aus expandiertem Polytetrafluorethylen besteht.

4.  Verband nach einem vorhergehenden Anspruch, bei dem die Manschette (2) dadurch aus einem Membran-Blattmaterial gebildet ist, daß benachbarte Ränder des Blatts durch Heißsiegeln verbunden sind.

5.  Verband nach einem vorhergehenden Anspruch, bei dem die unbeweglich-machende Schicht (1) eine oder mehrere Schichten aus Gipsverbandmaterial aufweist.

6.  Verband nach einem der Ansprüche 1 bis 4, bei dem die unbeweglich-machende Schicht (1) ein organisches polymeres Material aufweist, welches ein Verstärkungsmaterial enthält.

7.  Verband nach Anspruch 6, bei dem das Verstärkungsmaterial Glasfasern oder organische Polymerfasern aufweist.

# FIG. I.

EP 0 289 681 B1